# EUROPEAN PATENT APPLICATION

(11) **EP 4 721 750 A1**
(43) Date of publication of application: **08.04.2026**
(21) Application number: 24425047.8
(22) Date of filing: 03.10.2024
(51) Int. Cl.: A61K 35/741, A61P 35/00, A61K 31/733, A61K 35/745, A61K 39/00, C07K 16/00

(54) **NUTRACEUTICAL FORMULATION FOR USE IN CANCER IMMUNOTHERAPY**

(71) Applicant: Biofarma S.r.l., 33036 Mereto di Tomba (UD) (IT); A-Mansia Biotech S.A., 1435 Mont-Saint-Guibert (BE); Centro Sperimentale del Latte S.r.l., 26839 Zelo Buon Persico (LO) (IT)
(72) Inventor: Scarpa, Germano, Mereto di Tomba (UD) (IT); Willem, Cheers, Mont-Saint-Guibert (BE); Suenaert, Peter, Mont-Saint-Guibert (BE); M de Vos, Willem, Mont-Saint-Guibert (BE); Miorelli, Lorenzo, Zelo Buon Persico (LO) (IT); Martinez Ramos, Ines, Zelo Buon Persico (LO) (IT); Morisco, Filomena, Nola (NA) (IT)
(74) Representative: Petraz, Gilberto Luigi

(57) **Abstract**

Nutraceutical formulations, compositions, products and methods for use in cancer immunotherapy are described. Such formulations may comprise probiotics and may be used in cancer immunotherapy treatment method of a subject, possibly in combination with antibiotics and cancer drugs.

## Description

### FIELD OF THE INVENTION

Embodiments described herein refer to nutraceutical formulations, compositions, products and methods for use in cancer immunotherapy.

### BACKGROUND OF THE INVENTION

The immune system is the body's primary defense against infection and cancer. It is made up of a complex network of cells, molecules, organs and lymph tissues working together to defend the body against microorganisms such as bacteria, viruses and fungi, as well as against cancer cells. To be able to do this, the immune system must be able to distinguish between cells that naturally belong in the body (self) and foreign cells (non-self). Antigens are substances that the immune system recognizes as toxic and stimulate an immune response; in other words, they are non-self organisms. An antigen may be a substance from the environment (such as a bacterium, a virus, or pollen), or it could be formed inside the body (such as a cancer cell).

Many cancers are likely prevented by the immune system's ability to recognize and destroy abnormal cells before they become cancerous, but even a healthy immune system cannot always prevent cancers from forming. Some cancer cells are able to develop and grow even in the presence of a healthy immune system.

Immunotherapy can play an important role in this situation as it can seek to activate or reactivate the immune system to attack and destroy cancer cells that have escaped immune detection.

Immunotherapy is a treatment that uses a person's own immune system to fight cancer. Immunotherapy can boost or change how the immune system works so it can find and attack cancer cells. This therapy is generally provided to cancer patients to stop the spread of cancer, to slow down the proliferation of cancer cells, and to improve their natural immunity for destroying the cancer cells.

There are different types of immunotherapies. These include monoclonal antibodies, checkpoint inhibitors, and vaccines. Some types of immunotherapies are also called "targeted treatments" or biological therapies. The introduction of new immunotherapies-based therapies has radically changed the scenario of cancer treatment. Indeed, immune checkpoint inhibitors (ICIs) in monotherapy or in combination regimen have now become the first- or second-line therapies for a variety of cancers (Timeline of Progress in Immunotherapy, 2020). Considering that only a subset of patients will respond, and others will develop resistance during the treatment, the main goal of cancer research is currently to enhance their efficacy.

Immunotherapy drugs are called "Immune Checkpoint Inhibitors (ICIs)" which can be translated as inhibitors of the "blocking" molecules of the immune response, because they inhibit the action of the receptors that prevent T lymphocytes from destroying tumor cells. These "defend" themselves from T lymphocytes by means of a molecule present on their membrane, called PD-L 1 (Programmed death-ligand 1), which, by binding to PD-1 (Programmed cell death protein 1), tells the T lymphocyte not to attack them. In the field of oncology, various drugs have been developed that act on this mechanism.

These are monoclonal antibodies that bind to PD-1 or PD-L1, respectively. The outcome of both links is to prevent the interaction between PD-1 and PD-L1 from blocking T lymphocytes capable of attacking and eliminating cancer cells.

Dietary factors that affect the gut microbiome may influence response to immunotherapy in melanoma patients. The first clinical study to report on the relationship between diet, microbiome, and response to anti-programmed cell death-1 (PD-1) checkpoint inhibitors found melanoma patients who consumed a high-fiber diet had greater gut microbiome diversity and a better response to treatment (Spencer CN, 2021).

Interestingly, mounting evidence in animal and human studies suggests a crucial role of the gut microbiota on the efficacy of immunotherapies against different tumors. Indeed, recent studies (Routy B, 2018) (Gopalakrishnan V, 2018) revealed a different gut microbiome composition (especially *Akkermansia muciniphila, Ruminococcacae family, Clostridia, Firmicutes, Faecalibacterium*) between responders and non-responders to ICIs (Immune checkpoint inhibitors) and that antibiotics inhibited their clinical benefit. Interestingly specific species transplantation (*A. muciniphila*) or faecal microbiota transplantation (FMT) from cancer patients who responded to ICIs or specific into germ-free or antibiotic-treated mice ameliorated the antitumor effects of PD-1 blockade, whereas FMT from nonresponding patients failed to do so.

Based on these data, ongoing clinical trials have been recently designed to test the safety and efficacy of FMT from ICIs-responders into patients treated with ICIs. Of note, although FMT is a very attracting approach, it is still in experimental stage (type of delivery, selection of donor) and not totally free of adverse events.

Finally, although affected from potential biases (retrospective study, different tumors, and different ICIs etc), publication Pinato DJ, 2019 is of interest: the time point of antibiotic assumption was crucial in determining ICIs efficacy. Indeed, the authors reported that patients who received antibiotic treatment within 30 days before starting ICIs presented worse prognosis in comparison to those that did not or those who received antibiotics during ICIs treatment.

In the oncological field, immunotherapy (Finn RS, 2020) (Yau T, 2020) has been studied for several years in association with traditional therapeutic protocols such as chemotherapy and radiotherapy, but recent successes, such as the complete healing from disease, reveal a promising direction for the future. Most of these studies reveal how the intestinal microbiota activates the local immunity to potentiate the immunotherapy efficacy against cancer. Recent studies demonstrate the diversity and abundance of links between specific probiotic strains and the therapeutic results in blocking the PD-1/PD-L1 axis, acting at the cell membrane of activated lymphocytes T (CD8+, CD4+), lymphocytes B, monocytes, NK cells and Antigen Presenting Cells (APC), dendritic cells included.

The interaction between PD-1 and its ligands, PD-L1 and PD-L2, exerts a negative regulation effect on the T- and B-cells functions, bringing to the reduction of anti-tumoral and anti-inflammatory immune responses. An approach may be to establish the existence of a link between a specific microbiota strains composition and the possible cancer therapy potentiation. Recent studies identified *Akkermansia muciniphila* (Mager, Science) (Ottman N) as a promising species for tumor immunotherapy. It has been demonstrated that the presence of *Akkermansia muciniphila* in some patients enhances immunotherapy success. Moreover, this synergistic activity is demonstrated by studies conducted on animal models. In fact, mice models of colorectal and lung cancer treated with anti-CTLA4 and anti-PD1 antibodies displayed a consistent reduction of tumor mass following the coadministration of immunotherapeutic monoclonal antibodies and *Akkermansia muciniphila* and/or *Bifidobacterium spp* (al. W. L., 2020) (al. M. L., 2020) (Miller PL., 2020). This may be a predictive or prognostic factor of immunotherapy success against cancer, such as colorectal, lung cancer and other cancers such as those of the liver. The aim can be regarded as how to transform the microbiota in an oncological biomarker.

There is therefore the need to provide a specific probiotic therapy as immunotherapy adjuvant that can overcome at least one of the disadvantages of the state of the art.

One purpose of the present disclosure is therefore to provide nutraceutical formulations, compositions, products and methods for use in cancer immunotherapy.

The Applicant has devised, tested and embodied the present invention to overcome the shortcomings of the state of the art and to obtain these and other purposes and advantages.

### SUMMARY OF THE INVENTION

The present invention is set forth and characterized in the independent claim. The dependent claims describe other characteristics of the present invention or variants to the main inventive idea.

In accordance with the above purposes, a nutraceutical formulation including probiotics as an adjuvant to immunotherapy of cancers may be provided. This novel formulation may be useful as a support in cancer immunotherapy.

Embodiments described herein refer to a nutraceutical formulation for use in cancer immunotherapy comprising pasteurized *Akkermansia muciniphila* strain and a mixture of at least *Bifidobacterium adolescentis* and *Bifidobacterium longum* strains.

In some embodiments, the above formulation may further comprise one or more prebiotic sources, in particular dietary fibers, more particularly inulin.

In further embodiments, the above formulation may be in the form of an orosoluble formulation.

In still further embodiments, the above formulation may be used in an immunotherapy method that involves administration to a patient of said formulation and of a cancer drug suitable for immunotherapy, in particular at least one monoclonal antibody, more particularly a combination of at least two monoclonal antibodies.

In yet further embodiments, the above formulation may be used in an immunotherapy method that involves administering said formulation to said subject prior to administering said at least one cancer drug suitable for immunotherapy, in particular in a range of 1 to 10 days, particularly 2 to 7 days, more particularly 3 to 6 days, before administering said at least one cancer drug suitable for immunotherapy.

In yet further embodiments, the above formulation may be used in an immunotherapy method that involves administering to said subject at least one antibiotic, in particular a mixture of at least two antibiotics, prior to administration to said patient of said formulation and of at least one cancer drug suitable for immunotherapy.

In still further embodiment, the above formulation may be used in an immunotherapy method that involves administering said at least one antibiotic to said subject prior to administering said formulation, in particular in a range of 1 to 35 days, particularly 1 to 30 days, more particularly 1 to 25 days, before administering said formulation.

Further embodiments described herein refer to a packaged product comprising a formulation according to embodiments of the present disclosure.

A further aspect of the present disclosure may refer to a therapeutic kit for use in cancer immunotherapy comprising a formulation according to the present disclosure, at least one antibiotic, in particular a mixture of at least two antibiotics, and at least one cancer drug suitable for immunotherapy, in particular at least one monoclonal antibody, more particularly a combination of at least two monoclonal antibodies.

Other aspects described herein may refer to probiotic formulations, such as for instance the above described formulations, for use in a cancer immunotherapy treatment method of a subject, said method comprising administering to said subject at least one antibiotic, in particular a mixture of at least two antibiotics, then administering to said subject said probiotic formulations and then administering to said subject at least one cancer drug suitable for immunotherapy, in particular at least one monoclonal antibody, more particularly a combination of at least two monoclonal antibodies.

### DETAILED DESCRIPTION OF EMBODIMENTS

It shall be clarified that the phraseology and terminology used in the present description, as well as the figures in the attached drawings also in relation as to how described, have the sole function of better illustrating and explaining the present invention, their purpose being to provide a non-limiting example of the invention itself, since the scope of protection is defined by the claims.

It is understood that elements and characteristics of one embodiment can be conveniently combined or incorporated into other embodiments without further clarifications.

Before describing these embodiments, it shall be also clarified that the present description is not limited in its application to details of the construction and disposition of the components as described in the following description using the attached drawings. The present description can provide other embodiments and can be obtained or executed in various other ways. It shall also be clarified that the phraseology and terminology used here is for the purposes of description only, and cannot be considered as limitative.

All the percentages and ratios indicated refer to the weight of the total composition (for example indicated as % w/w), unless otherwise indicated. All the measurements are made, unless otherwise indicated, at 25°C and atmospheric pressure. All the temperatures, unless otherwise indicated, are expressed in degrees Centigrade.

All the ranges reported here shall be understood to include the extremes, including those that report an interval "between" two values. Furthermore, all the ranges reported here shall be understood to include and describe the punctual values included therein, and also all the sub-intervals. Moreover, all the ranges are intended as such that the sum of the values comprised therein, in the final composition, gives 100%, in particular considering that the person of skill will know how to choose the values of the ranges so that the sum does not exceed 100%.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

Embodiments described herein refer to a nutraceutical formulation for use in cancer immunotherapy comprising pasteurized (i.e., heat killed or heat treated) *Akkermansia muciniphila* strain and a mixture of at least *Bifidobacterium adolescentis* and *Bifidobacterium longum* strains.

Pasteurized *Akkermansia muciniphila* as used in the embodiments described herein may be a novel food consisting of pasteurised (i.e., heat treated) *Akkermansia muciniphila.* This novel food is the subject of an application for authorisation of pasteurised *Akkermansia muciniphila* for use in food supplements and in foods for special medical purposes in the European Union (EU). It may be available from A-Mansia Biotech S.A., Belgium.

*Akkermansia muciniphila* is a human gut commensal non-motile and non-spore-forming elliptical bacterium, accounting for 1 to 5% of healthy intestinal microbiota. The strain *Akkermansia muciniphila* MucT (=ATCC BAA-835) was isolated from the intestinal tract of a healthy human.

This bacterium, a "good" component of intestinal microbiome, could prove to be very useful against aggressive tumors thanks to its ability to boost immune cells. The present disclosure provides such pasteurized *Akkermansia muciniphila* as an adjuvant treatment to immunotherapy in cancers such as for instance hepatocellular carcinoma.

The mechanism of action of this probiotic strain lies on the instauration of a physical interaction between a transmembrane protein known as Amuc _1100 and the intestinal component of the immune system. Amuc_1 100 can interact with Toll-like Receptor 2 (TLR-2). This interaction activates the TLR-2-associated signalling pathway, which leads to the production of interleukins and macrophages.

*Bifidobacterium adolescentis* and *Bifidobacterium longum* as used in the embodiments described herein are strains capable of producing acetates which can be converted to butyrate, the most important metabolite secreted in the colon. Moreover, these strains produce a metabolite called inosine, which is produced by nucleotides catabolism. It has been observed that this molecule is able to promote TH-1 lymphocytes activation, predominantly involved in defense against intracellular pathogens (e.g., viruses) and antitumor activity to increase the secretion of interferon gamma (IFN-g).

Overall, the presence of pasteurized *Akkermansia muciniphila* strain and *Bifidobacterium adolescentis* and *Bifidobacterium longum* provide a stimulating effect on the immune system, which can be very useful to support cancer drug therapy.

In some embodiments, the above formulation may further comprise one or more prebiotic sources, in particular dietary fibers, more particularly inulin.

Inulin is a dietary fiber that stimulates anti-tumor immunity in mice with melanoma, thus helping to slow down cell proliferation. It has been shown that the administration of inulin can modify the intestinal microbiota, favoring the growth of bacteria that help the immune system fight against cancer. Prebiotics could represent a powerful tool to restructure the gut microbiota and to identify bacteria that contribute to antitumor immunity (Li Y, 2020).

In still further embodiments, the above formulation may be used in an immunotherapy method that involves administration to a patient of said formulation and of a cancer drug suitable for immunotherapy, in particular at least one monoclonal antibody, more particularly a combination of at least two monoclonal antibodies.

In yet further embodiments, the above formulation may be used in an immunotherapy method that involves administering said formulation to said subject prior to administering said at least one cancer drug suitable for immunotherapy, in particular in a range of 1 to 10 days, particularly 2 to 7 days, more particularly 3 to 6 days, before administering said at least one cancer drug suitable for immunotherapy. Examples may be 2, 3, 4, 5, 6 and even more than 6 days, for instance up to 10 days before.

In yet further embodiments, the above formulation may be used in an immunotherapy method that involves administering to said subject at least one antibiotic, in particular a mixture of at least two antibiotics, prior to administration to said patient of said formulation and of at least one cancer drug suitable for immunotherapy.

In still further embodiment, the above formulation may be used in an immunotherapy method that involves administering said at least one antibiotic to said subject prior to administering said formulation, in particular in a range of 1 to 35 days, particularly 1 to 30 days, more particularly 1 to 25 days, before administering said formulation. Examples may be 20, 21, 22, 23, 24, 25 and even more than 25 days, for instance up to 30 or 35 days before.

Formulations described herein, therefore, may be nutraceutical compositions. The term "nutraceutical composition" as used herein is intended to include a functional food composition suitable for administration to a subject, such as a mammal, especially a human.

In embodiments, formulations described herein may include a food grade carrier.

In one embodiment, one or more of the strains described herein may be conjugated to the at least one food grade carrier. In another embodiment, one or more of the strains described herein may be encapsulated or associated with the at least one food grade carrier.

In a particular embodiment, the at least one food grade carrier may be selected from the group consisting of carbohydrates, lipids, peptides, proteins or combinations thereof.

Formulations suitable for the purposes described here can be liquid, solid, semisolid, powder.

In further embodiments, the above formulation may be in the form of an orosoluble formulation. Other form of the formulation may be contemplated.

Further embodiments described herein refer to a packaged product comprising a formulation according to embodiments of the present disclosure.

Formulations described herein may be packaged as such or mixed with acceptable excipients and conveniently formulated in any form suitable for enteral or oral administration, including solid forms such as powders, granules, sachets, stick-packs, flow-packs, capsules, tablets possibly with normal or controlled release, for example time or pH dependent, or filmed, or gastro-protected, with colon-specific or multilayer release, chewing gum or sweets, or liquid forms such as syrups, drops, elixirs, solutions and suspensions in type.

For example, compositions described herein may be mixed with acceptable excipients and conveniently formulated for use in any form suitable for the intended use.

The formulations described herein may be specifically formulated for administration by any suitable route, in particular oral administration (including buccal and sublingual).

### EXPERIMENTAL SECTION

### 1) In vitro experimentation: Effect of Akkermansia municiphila MucT heat treated, i.e., pasteurized, alone and in association with both Bifidobacterium longum SP54 DSM 29470 and Bifidobacterium adolescentis SP77 DSM 35140 on murine hepatoma Hepa 1-6 cells viability

The starting aim of this set of *in vitro* experiments was to assess the effect on murine hepatoma Hepa 1-6 cell line viability of different concentrations of *Akkermansia municiphila* MucT heat treated (defined as pAKK) and *Akkermansia municiphila* MucT pasteurized (i.e., heat treated) + *Bifidobacterium longum* SP54 DSM 29470 and *Bifidobacterium adolescentis* SP77 DSM 35140 at several experimental times by MTT assay. Formulations including *Akkermansia muciniphila* strain and a mixture of *Bifidobacterium adolescentis* and *Bifidobacterium longum* strains may be defined here and in the present disclosure as pAKK+Bifido formulations or simply pAKK+Bifido.

### Materials and Methods

### Cell culture

Authenticated Hepa 1-6 (ATCC number CCL-CRL-1830) was bought from the American Type Culture Collection (Manassas, VA, USA), amplificated by and stored in CEINGE Biotecnologie Avanzate Franco Salvatore cell bank (Naples, Italy).

Cells were cultured in Dulbecco's Modified Eagle Medium (DMEM) with 10% fetal bovine serum (FBS), supplemented only with 2 mM L-glutamine and maintained at 37°C in a humidified incubator under 5% CO2 and 95% air. All products used to culture cells were purchased from Invitrogen (Carlsbad, CA, USA).

### Determination of cell viability by MTT assay

Cell viability was evaluated by 3-(4,5-dimethylthiazol-2-yl)-2,5-diphenyltetrazolium bromide (MTT) assay, based on the formation of formazan from MTT by metabolically active cells.

Hepa 1-6 (5000 cells/well) were seeded into 96-well plates in complete medium for 24 h. Then, cells were stimulated in DMEM with 2% FBS with *Akkermansia municiphila* MucT heat treated, i.e., pasteurized, (pAKK) (A, B, C, and D groups) and *Akkermansia municiphila* MucT heat treated + *Bifidobacterium longum* SP54 DSM 29470 and *Bifidobacterium adolescentis* SP77 DSM 35140 (pAKK+Bifido) (E, F, G, and H groups). The experimental groups were described as follows:
- Control (CTR): Hepa 1-6 in DMEM with FBS 2%;
- Group A: Hepa 1-6 with only pAKK 10^3 CFU;
- Group B: Hepa 1-6 with only pAKK 10^2 CFU;
- Group C: Hepa 1-6 with only pAKK 10^1 CFU;
- Group D: Hepa 1-6 with only pAKK 1 CFU;
- Group E: Hepa 1-6 with pAKK 10^3 CFU+ *B. longum* 10^11 and *B. adolescentis* 10^9 CFU;
- Group F: Hepa 1-6 with pAKK 10^2 CFU+ *B. longum* 10^10 and *B. adolescentis* 10^8 CFU;
- Group G: Hepa 1-6 with pAKK 10^1 CFU+ *B. longum* 10^9 and *B. adolescentis* 10^7 CFU;
- Group H: Hepa 1-6 with pAKK 1 CFU+ *B. longum* 10^8 and *B. adolescentis* 10^6 CFU.

All the dilutions reported above were made in DMEM with FBS 2% (according to serial dilutions from the starting concentrations reported in microbiological specification sections of datasheets).

After 12, 24, 48 and 72 h of products incubation at 37°C, MTT (Sigma-Aldrich) was added to the medium to a final concentration of 0,5mg/ml for 1 h. Thereafter, the resulting formazan crystals were dissolved in dimethyl sulfoxide and the absorbance was measured on a microplate reader (EnSpire Multimode Plate Reader, PerkinElmer, Waltham, MA, USA). The optical density (OD₄₈₀) of the samples treated were compared with the OD₄₈₀ of control wells (CTR) to assess the cell viability, which was calculated as: % viable cells = (OD treated/OD control) ×100.

### Statistical analysis

Data are presented as mean ± SEM. Statistical analysis was performed by analysis of variance test for multiple comparisons followed by Bonferroni's test, using GraphPad Prism 8 (GraphPad software, San Diego, CA, USA). Statistical significance was set at P < 0.05.

### Results

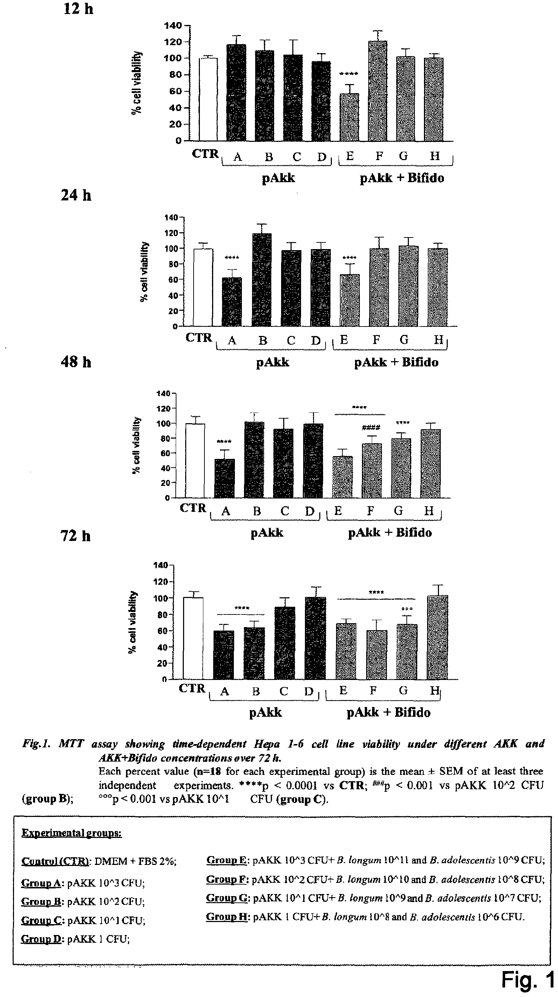

As reported in Figure 1, pAKK induces a significant decrease in cell viability already at 24 h at the highest dose (10^3 CFU) (group A) (****p < 0.0001 vs CTR). The effect is also observed for the second and the third highest dose used (group B and C), but only at 72 h (****p < 0.0001 vs CTR).

Even pAKK+Bifido induces significant decrease of cell viability at 24 h (group E) at the highest dose (10^3 CFU), but the effect is also observable for the other doses already at 48 h (group F) up to 72 h (group F and G) (****p < 0.0001 vs CTR) (Figure 1). Finally, at 12 h, a significant reduction in cell viability was observed only for pAKK+Bifido at the highest dose (10^3 CFU, group E) (****p < 0.0001 vs CTR) (Figure 1).

### 2) In vivo experimentation: modulation of the microbiome on the efficacy of immunotherapy in an orthotopic mouse model of hepatocellular carcinoma, by intrahepatic inoculation of Hepa 1-6 cells.

### Materials and Methods

Animal study (preclinical) has been carried out on 48 mice (C57BL/6J) of 6 week divided into 7 groups, after tumor cells inoculation with Hepa 1-6 cells - (5×10⁶/animal). Mice have been treated for 30 days and tumor growth was monitored every 4 days using ultrasonography:
• Group A: Amps/Strep (Antibiotic cocktails) + Bevacizumab + Atezolizumab (immunotherapies A+B b)
• Group B: Bevacizumab + Atezolizumab (immunotherapies A+B ^{b})
• Group C: Amps/Strep (Antibiotic cocktails) + formulation including pAKK+Bifido + Bevacizumab + Atezolizumab (immunotherapies A+B ^{b})

- Group D: Amps/Strep (Antibiotic cocktails) Internal Control
- Group E: formulation including pAKK+Bifido + Bevacizumab + Atezolizumab (immunotherapies A+B ^{b})
- Group F: Amps/Strep (Antibiotic cocktails) + formulation including pAKK+Bifido
- Group G: Amps/Strep (Antibiotic cocktails) +Akkermansia + Bevacizumab + Atezolizumab (immunotherapies A+B ^{b})

The radiologic assessment was made by ultrasound and magnetic resonance. The exploratory analysis included:
a. Faecal microbiome (Timepoints: before antibiotic treatment; b) at the end of antibiotic treatment; c) T0/T6; d) T5/T11; e) T15/T21.)
b. Tumor microbiome and mucosal microbiome (at sacrifice)
c. Immunological evaluation (before Hepa 1-6 injection; before immunotherapy treatment; 7 days after immunotherapy treatment start; at the end of immunotherapy treatment).

The end points evaluated for this *in vivo* experimentation are: evaluation of tumor number and dimensions, immunological profile, microbiota composition.

In the following Table 1, the treatment group scheme is explained.

| Table 1: treatment group scheme | | | | | | |
|---|---|---|---|---|---|---|
| Amps/Strep (Antibiotic cocktails Ampicillin and Streptomycin) | | | | | | |
| Atezolizumab + Bevacizumab (immunotherapies A+B ^{b}) | | | | | | |
| Formulation including pAKK+Bifido (*AkkermansiaA-Bifido*) | | | | | | |
| GROUP | Pretreatment Amps / Str /Col^{a} (Duration: 10 days) | Treatment 1 (after inoculation of Hepa cells 1-6) [T_{days}] | Treatment 2 (after inoculation of Hepa cells 1-6) [T_{days}] | Ultrasound [T_{days}] | MRI [T_{days}] | blood draws [T_{days}] |
| A (n=8) | Antibiotic cocktails | A+B^{b} [T_{0; 3; 6; 9; 12; 15}] | No | [T_{5; 10; 16}] | To be established | [Pre-inoculate Hepa 1-6; T_{0; 5; 15}] |
| B (n=8) | No | A+B^{b} [T_{0; 3; 6; 9; 12; 15}] | No | [T_{5; 10; 16}] | To be established | [Pre-inoculate Hepa 1-6; T_{0; 5; 15}] |
| C (n=8) | Antibiotic cocktails | Formulation including pAKK+Bifido [ ₀₋₅] | A+B^{b} [T_{6; 9; 12; 15; 18; 21}] | [T_{11; 16; 22}] | To be established | [Pre-inoculation Hepa 1-6; T_{6; 11; 21}] |
| D (n=8) Internal Control | Antibiotic cocktails | No | No | [T_{5; 10; 16}] | To be established | [Pre-inoculate Hepa 1-6; T_{0; 5; 15}] |
| E (n=8) _{.} | No | Formulation including pAKK+Bifido [T₀₋₅] | A+B^{b} [T_{6; 9; 12; 15; 18; 21}] | [T_{11; 16; 22}] | To be established | [Pre-inoculation Hepa 1-6; T_{6; 11; 21}] |
| F (n=8) | Antibiotic cocktails | Formulation including pAKK+Bifido [T₀₋₅] | No | [T_{5; 10; 16}] | To be established | [Pre-inoculate Hepa 1-6; T_{0; 5; 15}] |
| G (n=8) | Antibiotic cocktails | Only *Akkermansia* [T₀₋₅] | A+B ^{b} [T_{6; 9; 12; 15; 18; 21}] | [T_{5; 10; 16}] | To be established | [Pre-inoculate Hepa 1-6; T_{0; 5; 15}] |

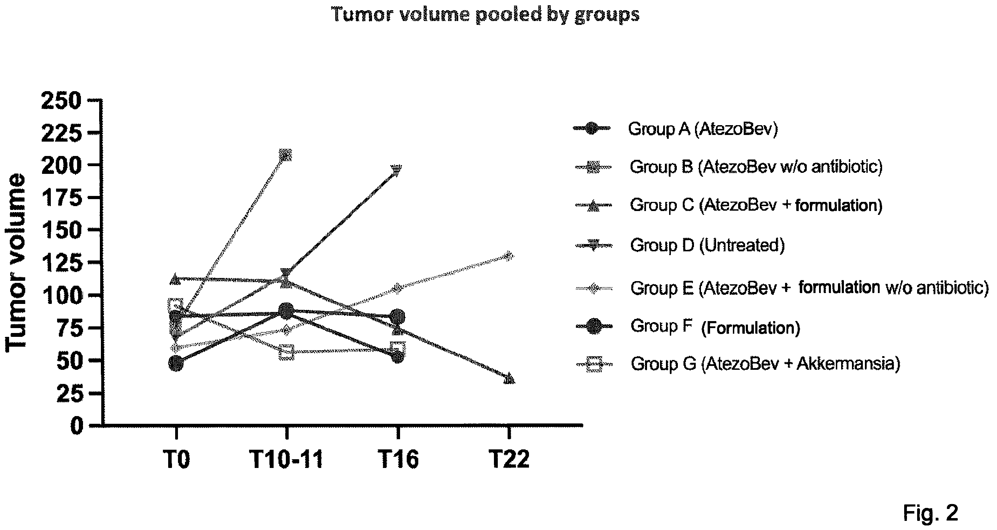

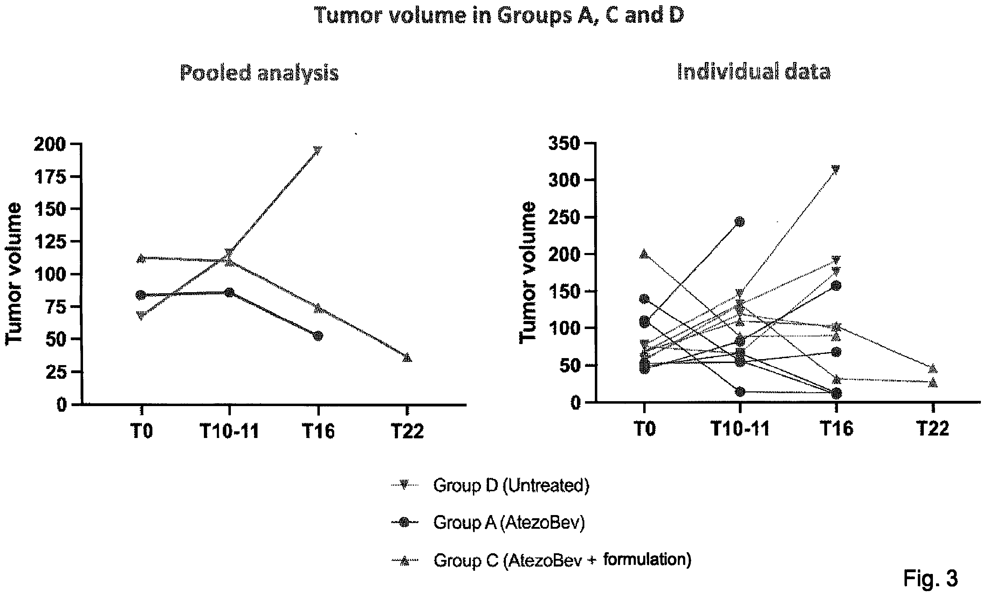

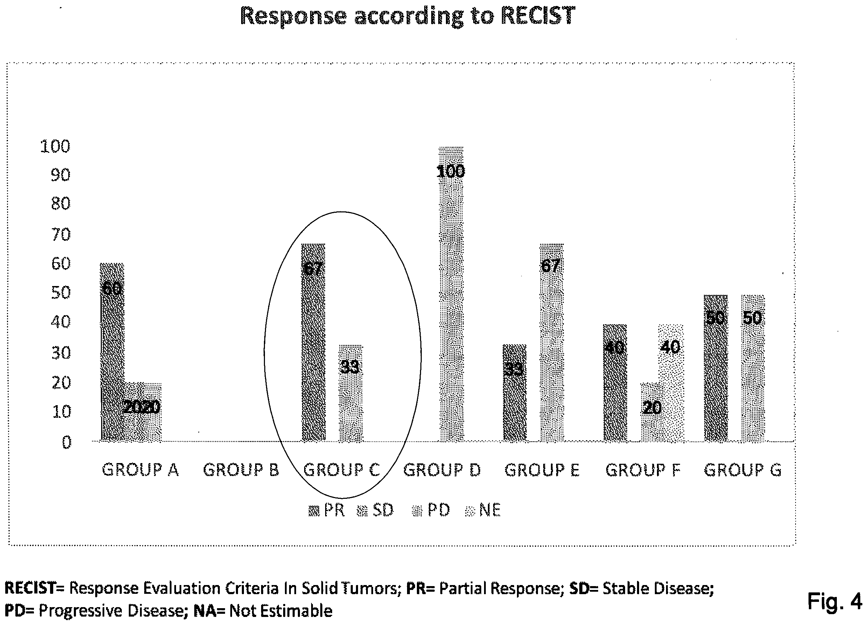

Data of tumor number and dimensions in mice are provided in Figures 2, 3 and 4. In particular Figure 2 shows a graph of tumor volume pooled within groups A to G and Figure 3 shows graphs of tumor pooled in groups A, C and D, respectively pooled analysis (left) and individual analysis (right). In Figures 2 and 3 the term "formulation" refers to formulation according to the present disclosure. Figure 4 shows a graph of response according to RECIST (Response Evaluation Criteria in Solid Tumors) for groups A to G.

The preliminary results of Figures 2, 3 and 4 show a tendency in the group of mice treated with antibiotics and immunotherapy (group A) to a slowdown in tumor growth, greater than in the group treated only with the immunotherapy (group B), indicating that there is likely a correlation between the microbiota composition and the effectiveness of pharmacological treatment.

It is interesting to observe that group C (circled in Figure 4), treated with antibiotic, the formulation including pAKK+Bifido according to the present disclosure and immunotherapy, appears to have an even more evident effect on slowing down the progression of tumor growth. This supports that by administering antibiotics to normalize the composition of the intestinal microbiota before starting the drug therapy and then by administering a blend with specific probiotics according to the formulation described herein, an intestinal environment favorable to a more effective immunotherapy treatment can be created. The development of an advanced treatment based on the association of these three phases can allow immunotherapy to be more effective, likely reducing non-responders, and to be able to last for a longer period.

These preliminary results show that the microbiota composition may influence the effectiveness of immunotherapy treatment.

The treatment with antibiotics is likely useful to normalize the microbiota composition at the beginning of the treatment, in order to prepare the microbiota to receive the strains that would appear useful to promote an increase in the effectiveness of immunotherapy. After this step, the administration of the formulation according to the present disclosure 5 days before the starting therapy could be able to introduce into the body the right strains, which can interact with the immune system and promote the immunotherapy.

The mechanism of action suggests that probiotics are indirectly implied in the stimulation of the immune system.

Without being bound by theory, the Applicant considers that this approach may be tumor independent and be rather linked to the immunotherapy mechanism of action and therefore can be used not only for the treatment of the hepatocellular carcinoma but for any type of tumor.

It is clear that modifications and/or additions of parts or steps may be made to nutraceutical formulations, compositions, products and methods for use in cancer immunotherapy as described heretofore, without departing from the field and scope of the present invention, as defined by the claims.

## Claims

1. Formulation for use in cancer immunotherapy comprising pasteurized *Akkermansia muciniphila* strain and a mixture of at least *Bifidobacterium adolescentis* and *Bifidobacterium longum* strains.

2. Formulation for use according to claim 1, further comprising one or more prebiotic sources, in particular dietary fibers, more particularly inulin.

3. Formulation for use according to claim 1 or 2 in the form of an orosoluble formulation.

4. Formulation for use according to claim 1, 2 or 3, wherein said use involves administration to a patient of said formulation and of at least one cancer drug suitable for immunotherapy, in particular at least one monoclonal antibody, more particularly a combination of at least two monoclonal antibodies.

5. Formulation for use according to claim 4, wherein said use involves administering said formulation to said subject prior to administering said at least one cancer drug suitable for immunotherapy, in particular in a range of 1 to 10 days, particularly 2 to 7 days, more particularly 3 to 6 days, before administering said at least one cancer drug suitable for immunotherapy.

6. Formulation for use according to claim 4 or 5, wherein said use involves administering to said subject at least one antibiotic, in particular a mixture of at least two antibiotics, prior to administration to said patient of said formulation and of at least one cancer drug suitable for immunotherapy.

7. Formulation for use according to claim 6, wherein said use involves administering said at least one antibiotic to said subject prior to administering said formulation, in particular in a range of 1 to 35 days, particularly 1 to 30 days, more particularly 1 to 25 days, before administering said formulation.

8. Packaged product comprising a formulation for use according to any claims hereinbefore provided in a suitable package.

9. A therapeutic kit for use in cancer immunotherapy comprising a formulation according to any claims from 1 to 7, at least one antibiotic, in particular a mixture of at least two antibiotics, and at least one cancer drug suitable for immunotherapy, in particular at least one monoclonal antibody, more particularly a combination of at least two monoclonal antibodies.

10. Probiotic formulation for use in a cancer immunotherapy treatment method of a subject, said method comprising administering to said subject at least one antibiotic, in particular a mixture of at least two antibiotics, then administering to said subject said probiotics and then administering to said subject at least one cancer drug suitable for immunotherapy, in particular at least one monoclonal antibody, more particularly a combination of at least two monoclonal antibodies.
